Europäisches Patentamt

European Patent Office

Office européen des brevets

(11) Publication number: **0 326 963 B1**

(12) **EUROPEAN PATENT SPECIFICATION**

(45) Date of publication of patent specification: **16.03.94**

(21) Application number: **89101461.5**

(22) Date of filing: **27.01.89**

(51) Int. Cl.⁵: **A61K 7/16**, A61K 7/32, A23L 1/211

(54) **Deodorant as well as deodorizing food, drink and seasoning.**

(30) Priority: **30.01.88 JP 19946/88**
**26.04.88 JP 101382/88**

(43) Date of publication of application:
**09.08.89 Bulletin 89/32**

(45) Publication of the grant of the patent:
**16.03.94 Bulletin 94/11**

(84) Designated Contracting States:
**AT BE CH DE ES FR GB IT LI NL SE**

(56) References cited:
**GB-A- 2 055 574**

**PATENT ABSTRACTS OF JAPAN, vol. 10, no. 136 (C-347)[2193], 20th May 1986; & JP-A-60 259 157 (ISAO SAKAI) 21-12-1985**

**PATENT ABSTRACTS OF JAPAN, vol. 6, no. 98 (C-106)[976], 8th June 1982; & JP-A-57 29 265 (ISAO SAKAI) 17-02-1982**

(73) Proprietor: **SANWA KAGAKU KENKYUSHO CO., LTD.**
**No. 35, Higashi-sotobori-cho**
**Higashi-ku Nagoya-shi Aichi-ken(JP)**

(72) Inventor: **Sawai, Kiichi c/o Sanwa Kagaku Kenkyusho Co.Ltd.**
**35, Higashi-sotobori-cho, Higashi-ku**
**Nagoya Aichi-ken(JP)**
Inventor: **Kurono, Masayasu c/o Sanwa Kagaku Kenkyusho Co.Ltd**
**35, Higashi-sotobori-cho, Higashi-ku**
**Nagoya Aichi-ken(JP)**
Inventor: **Asai, Hiromoto c/o Sanwa Kagaku Kenkyusho Co. Ltd.**
**35, Higashi-sotobori-cho, Higashi-ku**
**Nagoya Aichi-ken(JP)**
Inventor: **Mitani, Takahiko c/o Sanwa Kagaku Kenkyusho Co.Ltd**
**35, Higashi-sotobori-cho, Higashi-ku**
**Nagoya Aichi-ken(JP)**
Inventor: **Ninomiya, Naohisa Sanwa Kagaku Kenkyusho Co. Ltd.**
**35, Higashi-sotobori-cho, Higashi-ku**
**Nagoya Aichi-ken(JP)**
Inventor: **Furukawa, Eiji c/o Sanwa Kagaku Kenkyusho Co.Ltd**
**35, Higashi-sotobori-cho, Higashi-ku**
**Nagoya Aichi-ken(JP)**

Inventor: **Otsuka, Tamaki c/o Sanwa Kagaku Kenkyusho Co.Ltd**
**35, Higashi-sotobori-cho, Higashi-ku**
**Nagoya Aichi-ken(JP)**
Inventor: **Yamauchi Koji c/o Sanwa Kagaku Kenkyusho Co.Ltd**
**35, Higashi-sotobori-cho, Higashi-ku**
**Nagoya Aichi-ken(JP)**
Inventor: **Michishita, Hisashi c/o Sanwa Kagaku Kenkyusho Co.**
**Ltd. 35, Higashi-sotobori-cho, Higashi-ku**
**Sagoya Aichi-ken(JP)**


(74) Representative: **Wächtershäuser, Günter, Dr.**
**Tal 29**
**D-80331 München (DE)**

## Description

The present invention relates to a deodorant as well as a deodorizing food, drink and seasoning.

The deodorant is usually administered in oral route to substantially inhibit an appearance of various unpleasant or offensive smells such as ozostomy, body odor, smell of sweat, urogenous smell and others, or remarkably decrease the intensity of such smells.

Offensive smells should be excluded, so that a human communication shall not be impeded or to keep good life environment. Therefore, various studies have energetically been made to develop various types of deodorants, some of which have been put to practical use.

As conventional products for deodorizing the body odor, smell of sweat, excretions of man, pet animals, domestic animals and the like, there is one utilizing physical adsorption of the smells, another one causing decomposition of the smells contacting thereto, and the other one using a fragrant or aromatic substance to mask an offensive odor in question with its fragrant smell.

As the other type products for deodorizing ozostomy, there is one using chlorophyl, cinnamon, jasmine or the like fragrant substance, as effective ingredient, and one composing a germicidal substance to inhibit an activity of standing bacteria in oral cavity, which is one of the causes of generation of the ozostomy.

Each of the deodorants utilizing the physical adsorption of smell has the disadvantage of no immediate effect and a relatively low deodorizing capacity. While, each of the deodorants causing the decomposition of smell have also disadvantages that the decomposition accompanies eventually another unpleasant smell and that a sustaining effect can not be expected. In general, therefore, the deodorants utilizing the smell masking have widely been marketed. This type of deodorants shows drawbacks that although it has a tendency to select a fragrant substance with a stronger fragrance to effectively mask unpleasant smell(s), too strong fragrance may give a certain unpleasant feel to the third party, a smell depends on a personal liking or preference, so that the smell giving a nice feel to a person may give unpleasant feel to the third party, and there is such a case that a mixing of the fragrant masking smell with an odor to be masked shall often produce another unpleasant odor, even if temporary one.

GB-A-2055574 discloses an oral composition for caries prophylaxis which comprises stannous fluoride and a phytic acid compound, the molar ratio of the phytic acid compound to stannous fluoride being in the range from 0.01 to 4.1. The oral composition may be used as mouth washes, oral bands, topical solutions, and other dental purposes.

As referred to above, various deodorants have been proposed but those conventional products are not only show various disadvantages but also expect a passive action or effect thereof.

The inventors have found such a fact during nutritional physiological experiments on phytic acid and salts thereof that an administration of the compound or its salt makes gradually and substantially decrease various odors and more particularly ozostomy and smells of urine and sweat of experimented animals.

Turning now to the compound, phytic acid (mesoinosit-hexa-phosphate) has been known as a substance present in various plants in the form of calcium salt, magnesium salt or a mixed salt with potassium salt. The phytic acid, salts thereof and myosinositol which is one of concerned compounds with the phytic acid have been extracted from rice polishings (materials obtained when natural or unhulled rice is polished into cleaned one) which contain the phytic acid in an amount of 9.5 to 14.5% by weight. The phytic acid and salts thereof have been put into various uses. Namely, in medical field, calcium phytate has been used as one of calcium supply sources, an effective ingredient for curing carcemia or inhibiting calcariuria in sarcoidosis, and the rice polishings per se and sodium phytate has been employed as an effective ingredient for preventing a recidivation of calcium calculus. In general industrial fields, the phytic acid has been employed as a fermentation assisting agent in preparation of Japanese Sake and wines, a de-metal agent which utilizes a cheleting action of the acid, an anti-oxidant, anti-corrosion agent and others. Further, such a proposal has been made that a raw garlic is immersed in aqueous solution of phytic acid to deodorize an inherent smell thereof [Jap. Pat. No. 60 - 259157 (A) and JP-A-57 29265].

Hitherto, however, there has been issued no report to the effect that smells of body, urin and the like unpleasant one can be decreased by administration of phytic acid or a salt thereof to animals (please note that the phytic acid is one of strong acidic substances and each salt thereof is odorless one having pH in a range of 6 to 9).

A basic object of the invention is, therefore, to apply the phytic acid or a salt thereof for a deodorant, in view of its unique deodorizing action having been unexpectedly found during the course of said nutritional experiments.

A concrete object of the invention is to provide a new type deodorant which substantially decreases or inhibits an appearance of unpleasant smells, by mere oral dosage thereof.

Another concrete object of the invention is to provide a deodorant in the form of a food, drink or seasoning, so that an dosage or administration thereof is quite easy.

The above object is solved in accordance with the present invention by providing

a deodorant to be administrated by swallowing to inhibit an appearance of unpleasant smell, which comprises at least one compound selected from the group consisting of phytic acid and non-toxic salts thereof in an amount of 1 to 500 mg based on the phytic acid, in association with a non-toxic carrier.

Preferred embodiments of the invention are indicated in the subclaims.

The non-toxic salts of phytic acid mean those with a non-toxi metal, non-toxic organic base, basic amino acid, residue of organic ester or the like, and followings may be listed therefor.

Potassium phytate, sodium phytate, arginine phytate, ornithine phytate, lysine phytate, histidine phytate, glucamine phytate, monoethanolamine phytate, diethanolamine phytate, triethanolamine phytate.

As examples of unpleasant smells to be inhibited the following may be listed.

Ozostomy, body odor, smell of sweat, smell of excretions of pet animals and domestic animals; general ozostomy, ozostomies due to intake of foods with an intensive smell, such as garlic and leek, alcoholic drink, or smoking of tobacco, general body odor, smell of sweat inclusive of smells of tow armpit, inherent smell due to senility, fatty smell due to diseases, urogenous smell inclusive of smell of melituria, smell due to menstruation and the like of man.

A form of the deodorant according to the invention may be of a solution dissolved the phytic acid or salt thereof in water, powder to be prepared by adding an auxiliary and pulverizing in a conventional manner, or granule or tablet.

A dosing amount of the deodorant depends on kind and intensity of smell, form of the deodorant and other variable factors, but 1 - 500 mg/day and 1 - 15mg/kg/day are preferable for human adult and animals, respectively, based on phytic acid.

It is preferable to prepare various phytates having a pH range of 6 to 9 and to make same an effective ingredient in solely or in the form of a mixture, so that the effective ingredient may be selected in accordance with its use as a main ingredient for the deodorant or deodorizing additive for foods, drinks and seasonings, since the phytic acid is one of strong acids and the deodrant as well as other products according to the invention shall be administered in oral route. Following Table 1 shows molar amounts of various bases, which are required for converting 1 molar amount of phytic acid into phytinates having the pH range of 6 to 8.

Table 1

| Base | pH | | |
| --- | --- | --- | --- |
| | 6.00 | 7.00 | 8.00 |
| NaOH | 7.34 | 8.21 | 8.94 |
| KOH | 7.34 | 8.23 | 8.93 |
| LiOH | 7.41 | 8.38 | 9.30 |
| $HOCH_2CH_2NH_2$ | 7.72 | 8.68 | 9.52 |
| $(HOCH_2CH_2)_2NH$ | 7.54 | 8.68 | 9.31 |
| $(HOCH_2CH_2)_3N$ | 7.20 | 8.53 | 12.1 |
| N-Methylglucamine | 7.62 | 8.49 | 9.25 |
| L-Arginine | 7.79 | 8.67 | 9.60 |
| L-Lysine | 8.01 | 8.98 | 10.0 |
| L-Histidine | 11.3 | - | - |

The invention will now be further explained with reference to Examples and Test Examples.

Example 1

Potassium phytate was dissolved in purified water to prepare solutions, each containing potassium phytate in an amount of 8mg/ml and 20mg/ml, respectively.

Test Example 1

A solution containing potassium phytate in an amount of 8mg/ml (Example 1) was administered to cats (5 heads, body weight of about 5kg) in an amount of 3ml/head/day over 14 days. After 1, 3, 5, 7 and 14 days from the first administration, fresh urine was taken with use of a catheter to immediately check a degree of its smell by 3 judges. Results are shown in following Table 2. As seen from the Table, a deodorizing effect of the compound appears by third day and on fifth day, urogenous smell was disappeared. After lapsed several days from the final administration, urogenous smell was appeared again. After 10 days from the final administration, similar test was repeated to obtain results same with said first test.

Table 2

| No. | Class | Sex | Deodorizing Effects ( *) | | | | |
|---|---|---|---|---|---|---|---|
| | | | 1st day | 3rd day | 5th day | 7th day | 14th day |
| 1 | Mature | ♂ | 1 | 1 | 2 | 3 | 3 |
| 2 | Mature | ♂ | 1 | 2 | 3 | 3 | 3 |
| 3 | Mature | ♀ | 1 | 2 | 3 | 3 | 3 |
| 4 | Mature | ♀ | 1 | 2 | 3 | 3 | 3 |
| 5 | Youth | ♂ | 1 | 2 | 3 | 3 | 3 |
| Efficiency | | | 0/5 | 0/5 | 4/5 | 5/5 | 5/5 |

\* : 1 ; No change
2 ; Weaken
3 ; Almost no recognizable smell

Test Example 2

A solution containing potassium phytate in an amount of 20mg/ml (Example 1) was administered to crossbred mature cats (8 heads, body weight of about 5kg) in an amount of 3ml/head/day over 7 days. After 1, 3, 5 and 7 days from the first administration, fresh urine was taken with use of a catheter to immediately check a degree of its smell by 3 judges. Results are shown in following Table 3. As seen from the Table, a deodorizing effect of the compound appears by third day and on fifth day, urogenous smell disappeared.

Table 3

| No. | Sex | Deodorizing Effects ( *) | | | |
|---|---|---|---|---|---|
| | | 1st day | 3rd day | 5th day | 7th day |
| 1 | ♂ | 1 | 2 | 3 | 3 |
| 2 | ♂ | 1 | 2 | 3 | 3 |
| 3 | ♂ | 2 | 2 | 3 | 3 |
| 4 | ♂ | 1 | 2 | 3 | 3 |
| 5 | ♂ | 2 | 2 | 3 | 3 |
| 6 | ♀ | 1 | 2 | 3 | 3 |
| 7 | ♀ | 1 | 2 | 3 | 3 |
| 8 | ♀ | 1 | 2 | 3 | 3 |
| Efficiency | | 0/8 | 0/8 | 8/8 | 8/8 |

\* : 1 ; No change
2 ; Weaken
3 ; Almost no recognizable smell

Test Example 3

A powder composition was prepared by mixing potassium phytate and lactose, which composition containing the phytate in an amount of 24mg/g.

The composition was mixed with a powdered food which was intaken by crossbred mature cats (8 heads, body weight of about 5kg) in an amount of 1g/head/day based on the composition over 7 days. After 1, 3, 5 and 7 days from the first administration, fresh urine was taken with use of a catheter to immediately check a degree of its smell by 3 judges. Results are shown in following Table 4. As seen from the Table, a deodorizing effect of the compound appears by third day and on fifth day, urogenous smell disappeared.

Table 4

| No. | Sex | Deodorizing Effects ( *) | | | |
|---|---|---|---|---|---|
| | | 1st day | 3rd day | 5th day | 7th day |
| 1 | ♂ | 1 | 3 | 3 | 3 |
| 2 | ♂ | 1 | 2 | 3 | 3 |
| 3 | ♂ | 2 | 2 | 3 | 3 |
| 4 | ♂ | 1 | 3 | 3 | 3 |
| 5 | ♂ | 2 | 2 | 3 | 3 |
| 6 | ♀ | 1 | 2 | 3 | 3 |
| 7 | ♀ | 2 | 2 | 3 | 3 |
| 8 | ♀ | 2 | 2 | 3 | 3 |
| Efficiency | | 0/8 | 2/8 | 8/8 | 8/8 |

* : 1 ; No change
2 ; Weaken
3 ; Almost no recognizable smell

Test Example 4

To a moisture type cat food, potassium phytate was composed in an amount of 0.1mg/g.

The cat food was given to crossbred mature cats (2 heads, body weight of about 5kg) in an amount 100g/day over 7 days. After 2, 3, 4, 5 and 7 days from the first administration, fresh urine was taken with use of a catheter to immediately check a degree of its smell by 3 judges. Results are shown in following Table 5. As seen from the Table, a deodorizing effect of the compound appears by third day and on forth day, urogenous smell disappeared.

Table 5

| No. | Sex | Deodorizing Effects ( *) | | | | |
|---|---|---|---|---|---|---|
| | | 2nd day | 3rd day | 4th day | 5th day | 7th day |
| 1 | ♂ | 2 | 3 | 3 | 3 | 3 |
| 2 | ♂ | 2 | 2 | 3 | 3 | 3 |
| Efficiency | | 0/2 | 1/2 | 2/2 | 2/2 | 2/2 |

* : 1 ; No change
2 ; Weaken
3 ; Almost no recognizable smell

Test Example 5

An aqueous solution containing potassium phytate in an amount of 20mg/ml was added to 170 ml of drinking water for each experimented animal (rabbits, 2 heads) in amount of 2ml. The resulting drinking water was given for each day over 14 days, so that it can be intaken by the animal in free together with their food. After 1, 3, 5, 7 and 14 days from the first giving of the drinking water containing the compound, fresh urine was taken with use of a catheter to immediately check a degree of its smell by 3 judges. Results are shown in following Table 6. As seen from the Table, a deodorizing effect of the compound appears by third day and on fifth day, almost no unpleasant urogenous smell was notified. While, there was no change in a fragrant smell which may be considered due to plant terpenes contained in their food.

Table 6

| No. | Sex | Deodorizing Effects ( *) | | | | |
|---|---|---|---|---|---|---|
| | | 1st day | 3rd day | 5th day | 7th day | 14th day |
| 1 | ♂ | 1 | 2 | 3 | 3 | 3 |
| 2 | ♂ | 1 | 2 | 2 | 3 | 3 |
| 3 | ♀ | 1 | 1 | 3 | 3 | 3 |
| 4 | ♀ | 1 | 2 | 3 | 3 | 3 |
| Efficiency | | 0/4 | 0/4 | 3/4 | 4/4 | 4/4 |

* : 1 ; No change
2 ; Weaken
3 ; Almost no recognizable smell

Test Example 6

An aqueous solution containing potassium phytate in an amount of 20mg/ml was prepared. The solution was added to drinking water for each experimented animal (hamsters, 2 pairs) over 14 days, so that each animal can intake the compound in an amount of 10mg/kg. On each day during the testing period of time, urogenous smell was checked with use of finely cut newspaper which was employed instead of a litter to find that an intensity of the smell decreases on several days from the first administration of the compound containing drinking water.

Example 2 to 12 (First Pre-Compositions)

Following first pre-compositions were prepared.

First pre-composition A :

An aqueous solution prepared by adding 294g of sodium hydroxide to phytic acid of 660g based on its anhydride, and adding purified water with stirring to adjust pH of the resulting solution to 6.

First pre-composition B :

An aqueous solution prepared by adding 412g of potassium hydroxide to phytic acid of 660g based on its anhydride, and adding purified water with stirring to adjust pH of the resulting solution to 6.

First pre-composition C :

An aqueous solution prepared by adding 177g of lithium hydroxide to phytic acid of 660g based on its anhydride, and adding purified water with stirring to adjust pH of the resulting solution to 6.

First pre-composition D :

An aqueous solution prepared by adding 581g of ethanol amine to phytic acid of 660g based on its anhydride, and adding purified water with stirring to adjust pH of the resulting solution to 8.

First pre-composition E :

An aqueous solution prepared by adding 979g of diethanol amine to phytic acid of 660g based on its anhydride, and adding purified water with stirring to adjust pH of the resulting solution to 8.

First pre-composition F :

An aqueous solution prepared by adding 1805g of triethanol amine to phytic acid of 660g based on its anhydride, and adding purified water with stirring to adjust pH of the resulting solution to 8.

First pre-composition G :

An aqueous solution prepared by adding 1657g of N-methylglucamine to phytic acid of 660g based on its anhydride, and adding purified water with stirring to adjust pH of the resulting solution to 7.

First pre-composition H :

An aqueous solution prepared by adding 1510g of L-arginine to phytic acid of 660g based on its anhydride, and adding purified water with stirring to adjust pH of the resulting solution to 7.

First pre-composition I :

An aqueous solution prepared by adding 1171g of L-lysine to phytic acid of 660g based on its anhydride, and adding purified water with stirring to adjust pH of the resulting solution to 6.

First pre-composition J :

An aqueous solution prepared by adding 1753g of L-histidine to phytic acid of 660g based on its anhydride, and adding purified water with stirring to adjust pH of the resulting solution to 6.

First pre-composition K :

An aqueous solution prepared by adding 116g of sodium phytate, 478g of potassium hydroxide, 6.08g of calcium chloride (dihydrate) and 157g of disodium hydrogen phosphate (anhydride) to phytic acid of 660g based on its anhydride, and adding purified water with stirring to adjust pH of the resulting solution to 9.

Example 13 to 16 (Second Pre-Compositions)

Following second pre-compositions were prepared.

Second pre-composition A :

Powder prepared by taking the first pre-composition K (containing 200mg based on phytic acid), adding thereto lactose and drying to make into the powder (Total weight : 1000mg).

Second pre-composition B :

Powder prepared by taking the first pre-composition K (containing 100mg based on phytic acid), adding thereto lactose and drying to make into the powder (Total weight : 1000mg).

8

Second pre-composition C :

An aqueous solution prepared by taking the first pre-composition K (containing 100mg based on phytic acid), adding thereto purified water with stirring to make total weight into 1000mg.

Second pre-composition D :

An aqueous solution prepared by taking the first pre-composition K (containing 200mg based on phytic acid), adding thereto purified water with stirring to make total weight into 1000mg.

Example 17 (Elixir)

Following ingredients were composed to prepare clean transparent elixir in a conventional manner. A dosing amount of this elixir is 5ml containing 50mg based on phytic acid.

| | |
|---|---|
| Second pre-composition C | 100g |
| Orange extract (according to the Japanese Pharmacopeia) | 24ml |
| Ethanol | 400ml |
| Glycerine | 400ml |
| Purified water | remainder |
| | Total    1000ml |

Example 18 (Capsule)

Following ingredients were mixed and filled in a conventional manner to prepare capsules, each containing 40mg based on phytic acid.

| | |
|---|---|
| Second pre-composition A | 200mg |
| lactose | 20mg |
| Corn starch | 38mg |
| Magnesium stearate | 2mg |
| | Total    260mg/capsule |

Example 19 (Granule)

Following ingredients were composed and treated in a conventional manner to prepare granules which were sealed in packages. each containing 120mg based on phytic acid.

| | |
|---|---|
| Second pre-composition A | 600mg |
| lactose | 140mg |
| Corn starch | 250mg |
| Hydroxypropylcellulose | 10mg |
| | Total    1000mg/package |

Example 20 (Powder)

The second pre-composition A was packaged by aluminium seat seal packaging technique to accomodate the same by 1.5g, each package containing 300mg based on phytic acid.

Example 21 (Tablet)

Following ingredients were composed and treated in a conventional manner to prepare tablets having a diameter of 7mm, each containing 20mg based on phytic acid.

| Second pre-composition A | 100mg |
|---|---|
| Corn starch | 19mg |
| Crystalline cellulose | 30mg |
| Magnesium stearate | 1mg |
| | Total    150mg/tablet |

Example 22 (Syrup)

Following ingredients were dissolved and mixed to prepare cleanand transparent syrup. A dosing amount of syrup is 20ml containing 100mg based on phytic acid.

| Second pre-composition C | 50g |
|---|---|
| Refined sugar | 300g |
| D-Sorbitol (70%) | 250g |
| p-Oxymethyl benzoate | 0.3g |
| p-Oxypropyl benzoate | 0.15g |
| Sodium citrate | 10g |
| Citric acid | 1.5g |
| Flavour | 2g |
| Refined water | remainder |
| | Total    1000ml |

Example 23 (Dry Syrup)

Following ingredients were mixed and the mixture was wet-granuled with use of water and ethanol, in a conventional manner to prepre a dry syrup. Each package for this dry syrup contains 10mg based on phytic acid.

| Second pre-composition B | 100mg |
|---|---|
| Sodium citrate | 2.4mg |
| Citric anhydride | 2.2mg |
| Powdered tragacanth | 2.7mg |
| Refined sugar | suitable amount |
| Hydroxypropylcellulose | 3.0mg |
| Flavour | trace amount |
| | Total    1000mg/package |

Example 24 (Troche)

Under following weight ratio for ingredients, the second pre-composition A and refined sugar were wet-granulated with use of water and ethanol and dried at a temperature lower than 35°C. To the dry substance, lactose and magnesium stearate were mixed and tabletted to prepare troches having a diameter of 15mm, each containing 20mg based on phytic acid.

| Second pre-composition A | 100mg |
|---|---|
| Refined sugar | 870mg |
| Lactose | 20mg |
| Magnesium stearate | 10mg |
| | Total    1000mg/tablet |

Example 25 (Candy)

A heated mixture of refined sugar 240g, millet jelly 150g and refined water 100g was filtered to remove foreign materials and condensed in vacuo to prepare a candy texture at a temperature of 130 to 150°C and having a moisture content of 2 to 3%. To the texture, 10g of the second pre-composition B and a trace amount of a flavour were mixed. The mixture was charged in a mould to prepare candies, each containing 10mg based on phytic acid.

Example 26 (Drink)

Following ingredients were composed to prepare a drink. A dosing amount of this drink is 30ml containing 300mg based on phytic acid.

| Second pre-composition C | 3g |
|---|---|
| Simple syrup | 2.5ml |
| Refined water | remainder |
| | Total    30ml |

Example 27 (Granular Seasoning)

Following ingredient were mixed and wet-granulated with use of water and ethanol to prepare granular seasoning containing 100mg based on phytic acid.

| Second pre-composition D | 500mg |
|---|---|
| Garlic powder | 750mg |
| Lactose | remainder |
| | Total    1500mg |

Example 28 (Drink)

Following ingredients were composed to prepare clean and transparent drink. A dosing amount of this drink is 30ml containing 100mg based on phytic acid.

| Second pre-composition C | 1.0g |
|---|---|
| Honey | 0.5g |
| Refind sugar | 2.0g |
| Citric acid | suitable amount |
| Sodium citrate | ditto |
| Flavour (peppermint) | ditto |
| Refined water | remainder |
| | Total    30ml |

Example 29 (Garlic Seasoning)

With use of the following ingredient, a garlic seasoning was prepared in the manner similar to that in Example 27, which contains 100mg based on phytic acid.

| | | |
|---|---|---|
| Second pre-composition D | | 500mg |
| Abicel | | 180mg |
| Garlic powder | | 750mg |
| Lighter silicic anhydride | | 156mg |
| Corn starch | | 14mg |
| | Total | 1600mg |

Example 30 (Drink)

Following ingredients were composed to prepare a drink. A dosing amount of this drink is 20ml containing 100mg based on phytic acid.

| | | |
|---|---|---|
| Phytic acid | | 0.50g |
| Simple syrup | | 10.0ml |
| Refined water | remainder | |
| | Total | 100ml |

Test Example 7 (Stability Test)

Each of the products obtained by Examples 17 to 30 was accommodated in a glass bottle, a package of polyethyleneterephtharate or aluminum sheet, depending on the inherent form of the products, and then reserved for 3 weeks at 60°C. Thereafter, phytic acid content in each product was measured to find no decrease.

Test Example 8 (Inhibition of ozostmy)

(A) Testing Method

Following substances generating unpleasant smell were intaken by each panel (3 normal persons) who immediately drinks an aqueous solution of potassium phytinate (105mg/10ml). On contrary thereto, each control panel drinks were drinking water in same amount.

An inspection of ozostmy was carried out from time to time at intervals by 3 judges (to be judged as positive, when 2 or 3 judges feel the unpleasant smell) and by self notification of the experimented person.

Unpleasant smell generating materials :
a) Garlic (10g as grated one),
b) Leek (50g as cooked one),
c) Scallion (50g as pickled one),
d) Onion (100g as grated one),
e) Welsh onion (100g as cooked one),
f) Tobacco (carrying out a functional test by the third party on smokers who smoke more than 50 cigarette per diem), and
g) Alcohol (drink 300ml of Japanese sake, when the stomach of experimented person emties).

(B) Results

The judges declare that all of control groups are positive in each inspection carried out after 1, 3, 6, 12 and 24 hours from the intake, and that on contrary thereto, an ozostomy on the test group is not so strong already at the the time of first inspection carried out after 1 hour from the intake and apparently decreases at the time of second inspection carried out after 3 hours from the intake.

Similar results were obtained in self-judgement reported by the experimented persons.

Further, some of the control groups appeal a nausea, feel on foreign smell and abnormality on sence of smell, but nobody appeals such abnormalities in the test groups.

Test Example 9

The capsules obtained by Example 18 were given to volunteers who emit foreign smells due to hyperhidrosis (each 3 persons with toe smell and axillary odor, respectively) to orally administrate the same by one capsule after each time of eating (120mg/day based on phytic acid). The capsule was continuously administrated over 1 week and a functional test was carried out by 3 judges and by self-judgement.

All of the judges declare that the symptoms gradually disappear. Similar results were obtained also in self-judgement reported by the experimented persons.

Test Example 10

As experimental animal, rats were employed, they had been fed with a cat food of high protein content (30%) to excrete urine with a smell inherent in that of cats. To test group I, potassium phytate was administrated in abdominal cavity at a dose of 3mg/day over 5 days and while, to test group II, the compound was given in the similar to the above but at a dose of 30mg/day. To control group, no compound was given. A urogenous smell was checked to obtain results shown in following Table 7.

Table 7

| | Urogenous Smell (*) | | | |
| --- | --- | --- | --- | --- |
| | Before dose | 3rd day | 6th day | 9th day |
| Control group | + + | + + | + + | + + |
| Test group I | + + | + | ± | + |
| II | + + | ± | ± | + |

\* : Judged by 4 stage judgement of + +, +,  
± and −

Test Example 11 (liking)

A beef fillet was spiced with use of 15g of the garlic seasoning obtained by Example 27 (containing 1g based on phytic acid) as well as suitable amount of table salt and pepper and then cooked as beef steak to make it a test sample. As control sample, another beef fillet was spiced with use of a conventional garlic seasoning, table salt and pepper and cooked into beef steak.

Each of the test and control samples was given by panelars (20 members) to check liking thereof. Results are shown in following Table 8.

Table 8

|  | Same with Control | Better than Control | Worse than Control |
|---|---|---|---|
| Taste | 6 | 14 | 0 |
| Smell | 1 | 19 | 0 |

## Claims
## Claims for the following Contracting States : BE, CH, LI, DE, FR, GB, IT, NL, SE

1. A deodorant to be administrated by swallowing to inhibit an appearance of unpleasant smell, which comprises at least one compound selected from the group consisting of phytic acid and non-toxic salts thereof in an amount of 1 to 500 mg based on the phytic acid, in association with a non-toxic carrier.

2. A deodorant as claimed in claim 1, wherein said salt of phytic acid is selected from the group consisting of potassium phytate, sodium phytate, arginine phytate, ornithine phytate, lysine phytate, histidine phytate, glucamine phytate, monoethanolamine phytate, diethanolamine phytate and triethanolamine phytate.

3. A deodorant as claimed in claim 1, wherein said deodorant is formulated for being swallowed by human beings to inhibit an appearance of at least one unpleasant smell selected from the group consisting of ozostomy, body odor, smell of sweat, inherent smell due to senility, fatty smell due to diseases, urogenous smell and smell due to menstruation.

4. A deodorant as claimed in claim 1, wherein said deodorant is formulated for being swallowed by pet animals to inhibit an appearance of at least one unpleasant smell selected from the group consisting of ozostomy, body odor and excretions.

5. A deodorant as claimed in claim 5, wherein said formulated deodorant is made so as to administrate the same in an amount of 1 to 15 mg/kg/day.

6. A deodorant as claimed in claim 1, wherein said non-toxic carrier is selected from food and drinks.

7. A deodorant according to claim 1, wherein said non-toxic carrier is a seasoning.

## Claims for the following Contracting States : ES, AT

1. A deodorant composition to be administrated by swallowing to inhibit an appearance of unpleasant smell, characterized because it comprises:
   (A) at least one compound selected from the group consisting of phytic acid and non-toxic salts thereof in an amount of 1 to 500 mg. based on the phytic acid;
   (B) a non-toxic carrier, selected from among water and a solid product or liquid that can be eaten or drunk by humans or animals.

2. A deodorant composition as claimed in claim 1, characterized in that said salt of phytic acid is selected from the group consisting of potassium phytate, sodium phytate, arginine phytate, ornithine phytate, lysine phytate, histidine phytate, glucamine phytate, monoethanolamine phytate, diethanolamine phytate and triethanolamine phytate.

3. A deodorant composition as claimed in claim 1, wherein said deodorant composition is formulated for being swallowed by human beings to inhibit an appearance of at least one unpleasant smell selected from the group consisting of ozostomy, body odor, smell of sweat, inherent smell due to senility, fatty smell due to diseases, urogenous smell and smell due to menstruation.

**4.** A deodorant composition as claimed in claim 1, characterized because said deodorant composition is formulated for being swallowed by pet animals to inhibit an appearance of at least one unpleasant smell selected from the group consisting of ozostomy, body odor and excretions.

**5.** A deodorant composition, as claimed in claim 1, charcterized because it is formulated in such a way that it can be administrated in an amount of 1 to 15 mg/kg/day.

**6.** A deodorant composition, as claimed in claim 1, characterized because said non-toxic carrier is selected from among food and drinks.

**7.** A deodorant composition, as claimed in claim 1, charcterized because said non-toxic carrier is seasoning.

**Patentansprüche**

**Patentansprüche für folgende Vertragsstaaten : BE, CH, LI, DE, FR, GB, IT, NL, SE**

**1.** Ein durch Verschlucken zu verabreichendes Deodorant zur Verhinderung des Auftretens von unangenehmem Geruch, welches mindestens eine der aus der aus Phytinsäure und deren nicht-toxischen Salzen gebildeten Gruppe ausgewählte Verbindung in einer Menge von 1 bis 500 mg, bezogen auf Phytinsäure, zusammen mit einem nicht-toxischen Träger umfaßt.

**2.** Ein Deodorant gemäß Anspruch 1, worin das Salz der Phytinsäure aus der aus Kaliumphytat, Natriumphytat, Argininphytat, Ornithinphytat, Lysinphytat, Histidinphytat, Glucaminphytat, Monoethanolaminphytat, Diethanolaminphytat und Triethanolaminphytat bestehenden Gruppe ausgewählt ist.

**3.** Ein Deodorant gemäß Anspruch 1, worin das Deodorant zum Verschlucken durch menschliche Wesen formuliert ist, um das Auftreten von zumindest einem aus der aus Mundgeruch, Körpergeruch, Schweißgeruch, Eigengeruch aufgrund von Senilität, Fettgeruch aufgrund von Krankheiten, urogenem Geruch und Menstruationsgeruch bestehenden Gruppe ausgewählten unangenehmen Geruch zu verhindern.

**4.** Ein Deodorant gemäß Anspruch 1, worin das Deodorant zum Verschlucken durch Streicheltiere formuliert ist, um das Auftreten von zumindest einem aus der aus Mundgeruch, Körpergeruch und Ausscheidungen bestehenden Gruppe ausgewählten unangenehmen Geruch zu verhindern.

**5.** Ein Deodorant gemäß Anspruch 1, worin das formulierte Deodorant so hergestellt ist, daß es in einer Menge von 1 bis 15 mg/kg/Tag zu verabreichen ist.

**6.** Ein Deodorant gemäß Anspruch 1, worin der nicht-toxische Träger aus Lebensmitteln und Getränken ausgewählt ist.

**7.** Ein Deodorant gemäß Anspruch 1, worin der nicht-toxische Träger ein Gewürzmittel ist.

**Patentansprüche für folgende Vertragsstaaten : ES, AT**

**1.** Eine durch Verschlucken zu verabreichende Deodorantzusammensetzung zur Verhinderung des Auftretens von unangenehmem Geruch, dadurch gekennzeichnet, daß diese umfaßt:
(A) mindestens eine aus der aus Phytinsäure und deren nicht-toxischen Salzen gebildeten Gruppe ausgewählte Verbindung in einer Menge von 1 bis 500 mg, bezogen auf Phytinsäure;
(B) einen nicht-toxischen Träger, der aus Wasser und einem festen Produkt oder einer Flüssigkeit besteht, der von Menschen oder Tieren gegessen oder getrunken werden kann.

**2.** Eine Deodorantzusammensetzung gemäß Anspruch 1, dadurch gekennzeichnet, daß das Salz der Phytinsäure aus der aus Kaliumphytat, Natriumphytat, Argininphytat, Ornithinphytat, Lysinphytat, Histidinphytat, Glucaminphytat, Monoethanolaminphytat, Diethanolaminphytat und Triethanolaminphytat bestehenden Gruppe ausgewählt ist.

**3.** Eine Deodorantzusammensetzung gemäß Anspruch 1, worin die Deodorantzusammensetzung zum Verschlucken durch menschliche Wesen formuliert ist, um das Auftreten von zumindest einem aus der aus Mundgeruch, Körpergeruch, Schweißgeruch, Eigengeruch aufgrund von Senilität, Fettgeruch aufgrund von Krankheiten, urogenem Geruch und Menstruationsgeruch bestehenden Gruppe ausgewählten unangenehmen Geruch zu verhindern.

**4.** Eine Deodorantzusammensetzung gemäß Anspruch 1, dadurch gekennzeichnet, daß die Deodorantzusammensetzung zum Verschlucken durch Streicheltiere formuliert ist, um das Auftreten von zumindest einem aus der aus Mundgeruch, Körpergeruch und Ausscheidungen bestehenden Gruppe ausgewählten unangenehmen Geruch zu verhindern.

**5.** Eine Deodorantzusammensetzung gemäß Anspruch 1, dadurch gekennzeichnet, daß diese so formuliert ist, daß sie in einer Menge von 1 bis 15 mg/kg/Tag verabreicht werden kann.

**6.** Eine Deodorantzusammensetzung gemäß Anspruch 1, dadurch gekennzeichnet, daß der nicht-toxische Träger aus Lebensmitteln und Getränken ausgewählt ist.

**7.** Eine Deodorantzusammensetzung gemäß Anspruch 1, dadurch gekennzeichnet, daß der nicht-toxische Träger ein Gewürzmittel ist.

**Revendications**
**Revendications pour les Etats contractants suivants : BE, CH, LI, DE, FR, GB, IT, NL, SE**

**1.** Déodorant à administrer par ingestion pour inhiber l'apparition d'une odeur déplaisante, comprenant au moins un composé choisi dans le groupe constitué par l'acide phytinique et ses sels non toxiques à raison de 1 à 500 mg sur la base de l'acide phytinique, en association avec un véhicule non toxique.

**2.** Déodorant selon la revendication 1, dans lequel ledit sel d'acide phytinique est choisi dans le groupe constitué du phytinate de potassium, du phytinate de sodium, du phytinate d'arginine, du phytinate d'ornithine, du phytinate de lysine, du phytinate d'histidine, du phytinate de glucamine, du phytinate de monoéthanolamine, du phytinate de diéthanolamine et du phytinate de triéthanolamine.

**3.** Déodorant selon la revendication 1, dans lequel ledit déodorant est formulé pour être ingéré par des êtres humains afin d'inhiber l'apparition d'au moins une odeur déplaisante choisie parmi l'ozostomie, les odeurs corporelles, l'odeur de sueur, l'odeur inhérente à la sénilité, l'odeur graisseuse due à des maladies, l'odeur d'urine et l'odeur due aux menstruations.

**4.** Déodorant selon la revendication 1, dans lequel ledit déodorant est formulé pour être ingéré par des animaux domestiques pour inhiber l'apparition d'au moins une odeur déplaisante choisie parmi l'ozostomie, les odeurs corporelles et les odeurs d'excrétions.

**5.** Déodorant selon la revendication 1, dans lequel ledit déodorant est formulé pour être administré à raison de 1 à 15 mg/kg/jour.

**6.** Déodorant selon la revendication 1, dans lequel ledit véhicule non toxique est choisi parmi les aliments et les boissons.

**7.** Déodorant selon la revendication 1, dans lequel ledit véhicule non toxique est un assaisonnement.

**Revendications pour les Etats contractants suivants : ES, AT**

**1.** Composition déodorante susceptible à administrer par ingestion pour inhiber l'apparition d'une odeur déplaisante, caractérisée en ce qu'elle comprend :
A) au moins un composé choisi dans le groupe constitué de l'acide phytinique et de ses sels non toxiques à raison de 1 à 500 mg sur la base de l'acide phytinique, et
B) un véhicule non toxique choisi parmi l'eau et un produit solide ou liquide qui peut être mangé ou bu par des humains ou des animaux.

**2.** Composition déodorante selon la revendication 1, caractérisée en ce que ledit sel d'acide phytinique est choisi dans le groupe constitué du phytinate de potassium, du phytinate de sodium, du phytinate d'arginine, du phytinate d'ornithine, du phytinate de lysine, du phytinate d'histidine, du phytinate de glucamine, du phytinate de monoéthanolamine, du phytinate de diéthanolamine et du phytinate de triéthanolamine.

**3.** Composition déodorante selon la revendication 1, caractérisée en ce que ladite composition déodorante est formulée pour être ingérée par des êtres humains afin d'inhiber l'apparition d'au moins une odeur déplaisante choisie parmi l'ozostomie, les odeurs corporelles, l'odeur de sueur, l'odeur inhérente à la sénilité, l'odeur graisseuse due à des maladies, l'odeur d'urine et l'odeur due aux menstruations.

**4.** Composition déodorante selon la revendication 1, caractérisée en ce que ladite composition déodorante est formulée pour être ingérée par des animaux domestiques pour inhiber l'apparition d'au moins une odeur déplaisante choisie parmi l'ozostomie, les odeurs corporelles et les odeurs d'excrétions.

**5.** Composition déodorante selon la revendication 1, caractérisée en ce que ladite composition déodorante est formulée pour être administrée à raison de 1 à 15 mg/kg/jour.

**6.** Composition déodorante selon la revendication 1, caractérisée en ce que ledit véhicule non toxique est choisi parmi les aliments et les boissons.

**7.** Composition déodorante selon la revendication 1, caractérisée en ce que lequel ledit véhicule non toxique est un assaisonnement.